# EUROPEAN PATENT SPECIFICATION

(11) **EP 4 601 625 B1**
(45) Date of publication and mention of the grant of the patent: **09.12.2026**
(21) Application number: 23794271.9
(22) Date of filing: 13.10.2023
(51) Int. Cl.: A61K 9/28, A61K 9/20

(54) **MODIFIED RELEASE PHARMACEUTICAL FORMULATIONS COMPRISING DEFERIPRONE**
PHARMAZEUTISCHE FORMULIERUNGEN MIT MODIFIZIERTER FREISETZUNG MIT DEFERIPRON
FORMULATIONS PHARMACEUTIQUES À LIBÉRATION MODIFIÉE COMPRENANT DE LA DÉFÉRIPRONE

(30) Priority: 14.10.2022 EP 22201701
(43) Date of publication of application: 20.08.2025
(73) Proprietor: Chiesi Farmaceutici S.p.A., 43122 Parma (IT)
(72) Inventor: GAZZANIGA, Andrea, 43122 Parma (IT); CEREA, Matteo, 43122 Parma (IT); FOPPOLI, Anastasia, 43122 Parma (IT); PERTILE, Marisa, 43122 Parma (IT)
(74) Representative: Chiesi Farmaceutici S.p.A.
(86) International application number: PCT/EP2023/078445
(87) International publication number: WO 2024/079303

(56) References cited:
- US-A1- 2019 117 581
- KEERTHI LEELA ET AL: "Pharmaceutical mini-tablets, its advantages, formulation possibilities and general evaluation aspects: A review", INT. J. PHARM. SCI. REV. RES. SEPTEMBER - OCTOBER INTERNATIONAL JOURNAL OF PHARMACEUTICAL SCIENCES REVIEW AND RESEARCH, 1 January 2014 (2014-01-01), pages 214 - 221, XP093029238, Retrieved from the Internet <URL:https://globalresearchonline.net/journalcontents/v28-1/40.pdf> [retrieved on 20230306]

## Description

### FIELD OF INVENTION

The invention relates to pharmaceutical formulations comprising the iron chelator deferiprone, as defined by the claims.

In particular the invention is directed to a modified- release formulation in form of minitablets, as defined by the claims, suitable for twice-a-day oral administration for the treatment of diseases which cause an overload of iron for example, thalassemia, sickle cell anemia, hemochromatosis, and myelodysplasia, or for the prevention and/or treatment of diseases which are caused by an overload of iron.

### BACKGROUND OF THE INVENTION

Deferiprone, also known as 3-hydroxy- 1,2-dimethylpyridin-4-one, is a bidentate ligand which binds to iron in a 3:1 molar ratio.

It is used in the treatment of generalized iron overload, particularly in conditions where frequent blood transfusions lead to iron overload including, e.g., thalassemia and Sickle Cell Disease.

The introduction of deferiprone in the current therapy has represented an important advancement as it decreases Liver Iron Concentration (LIC) and cardiac iron overload.

In particular, Maggio A et al. (Blood Cells Mol Dis. 2002, 28(2):196-198) and Galanello R et al. (Haematologica. 2006, 91(9):1241-1243) suggested that deferiprone monotherapy could be superior to deferoxamine monotherapy in improving myocardial siderosis and cardiac function.

With regards to safety, the most frequent adverse events are gastrointestinal disorders due to gastrointestinal irritation.

Such discomfort could cause patients to refrain from taking the medication, leading to a worsening of their condition. Other observed adverse events are musculoskeletal disorders (arthralgia), Alanine Aminotransferase (ALT) increase, agranulocytosis and neutropenia.

Agranulocytosis seems to be an idiosyncratic response and it is more frequent in the first year of treatment.

The incidence of neutropenia and agranulocytosis is stable and seems to be not related with dose (Hider RC et al N Engl J Med. 2018;379:2140-2150).

Deferiprone is endowed with a half-life of 2-3 hour, and an unpleasant bitter taste too.

Said drug is sold as Immediate Release (IR) 500 mg and 1000 mg tablets, as well as a 100 mg/ml liquid formulation, generally, under the trade name Ferriprox^{®}.

In view of its pharmacological and ADME profile, and to improve the compliance of the patients, recently, deferiprone has also been commercially launched as 1000 mg Delayed Release (DR) tablets for oral administration.

Said tablets are suitable for a twice daily administration being bioequivalent in the steady state to the same daily dose of an immediate release tablet administered three times daily.

Said DR tablets are also debossed with a score line, to make it easy for the patient to break the tablets into two approximately equal parts to guarantee dosing flexibility.

The composition of the DR tablets has been disclosed in WO 2019/0822128, and it comprises: (a) a core comprising the active pharmaceutical ingredient and a releasing controlling enteric polymer, and (b) an enteric coating.

Upon oral administration, the enteric coating makes the dissolution in the stomach negligible, and hence subsequent dissolution of the active substance at physiological weakly acidic to weakly alkaline pH (e.g., pH 4.5 to 8), which corresponds to dissolution in the duodenum to ileum, is facilitated.

To sustain the release, in the case of the marketed deferiprone product, as enteric polymer in the tablet core, hydroxypropyl methylcellulose acetate succinate (HPMC-AS), is used.

However, HPMC-AS has a pH-dependent solubility.

This could lead to a release influenced by the external environment that the unit must face during the transit along the regions in which the release of the active principle takes place, that are characterized by physiological fluids having different pH's, and hence making said release less predictable being at the mercy of random microenvironmental variation of the pH.

Furthermore, it is difficult for a tablet that is enteric coated being broken into two parts without destroying the delayed release feature. This is because the surface at the interface of a broken tablet is no longer protected by the enteric coating.

If the unprotected core disintegrates and/or dissolves quickly, the dissolution of the broken tablet in the stomach acid could be faster than the whole tablet, so that protection against gastric irritation will be partially lost and the broken tablet would no longer deliver the drug at the same rate of the whole tablet.

Therefore, it would be advantageous to provide a pharmaceutical formulation comprising deferiprone suitable for twice a day oral administration with great dosing flexibility, but without the aforementioned possible drawbacks.

The solution is provided by the present invention.

### SUMMARY OF THE INVENTION

In a first aspect, the invention provides a modified release enteric coated pharmaceutical formulation in form of minitablets for twice-a-day oral administration, wherein the core of the minitablet comprises deferiprone as active ingredient in an amount comprised between 74% and 87%, a glyceryl ester of a long fatty acid as modifying release agent in an amount comprised between 10% and 20%, a lubricant and/or a glidant in an amount of 1 to 6%, and optionally another excipient in an amount of 0 to 2%, all the amounts calculated by weight on the total weight of the uncoated formulation, wherein the enteric coating comprises a mixture of methacrylic acid - ethyl acrylate copolymer (1:1), a plasticizer.

In a second aspect the invention provides a process for the preparation of a coated deferiprone tablet as described above, said process comprising the following steps:
(i) mixing deferiprone with the modifying release agent and the optional excipients, if present, to form a mixture;
(ii) tableting the mixture of step i) to obtain compacts;
(iii) crushing the compacts through a granulator with a suitable screen size to obtain a granulate;
(iv) mixing the granulate obtained in step (iii) with the lubricant/glidant excipient to form a final mixture;
(v) compressing the final mixture obtained in step (iv) to form a minitablet; and
(vi) coating the minitablet and drying.

In a third aspect, the invention is directed to a container filled with the minitablets of the invention.

In a fourth aspect, the invention is directed to the claimed pharmaceutical composition for use for the treatment of diseases which cause an overload of iron, or for the prevention and/or treatment of diseases which are caused by an overload of iron.

In a fifth aspect, the invention is directed to the claimed pharmaceutical composition in the manufacture of a medicament for the treatment of diseases which cause an overload of iron, or for the prevention and/or treatment of diseases which are caused by an overload of iron.

In a sixth aspect, the invention refers to a method for the treatment of diseases which cause an overload of iron, or for the prevention and/or treatment of diseases which are caused by an overload of iron in a patient in a need thereof, said method comprising orally administering the claimed pharmaceutical composition.

### BRIEF DESCRIPTION OF THE DRAWINGS

**Figure** 1- Dissolution profiles of deferiprone as such and from uncoated minitablets comprising different modifying release agents.
**Figure** 2- Dissolution profiles of deferiprone from reference commercial product and enteric coated minitablets

### DEFINITIONS

As used herein, the indefinite articles "a" or "an" should be understood to refer to "one or more" of any recited or enumerated component. For example, "a tablet" refers to one or more tablets.

Also as used herein, "and/or" refers to and encompasses all possible combinations of one or more of the associated listed items, as well as the lack of combinations when interpreted in the alternative ("or").

When the term "about" is used in conjunction with a numerical value or range, it modifies that value or range by extending the boundaries above and below the numerical values set forth. The term "about" is used herein to modify a numerical value above and below the stated value by a variance of 10 percent, up or down (higher or lower), i.e., ± 10%, unless a different variance is indicated (e.g., ± 30%, ± 20%, ± 5%, ± 1%, etc.).

Wherever aspects are described herein with the language "comprising," otherwise analogous aspects described in terms of "consisting of and/or "consisting essentially of are also provided. To the extent that the term "includes" or "including" is used in the specification or the claims, it is intended to be inclusive in a manner similar to the term "comprising" as that term is interpreted when employed as a transitional word in a claim.

As used herein, the term "active ingredient" or "active pharmaceutical ingredient" (API) or "drug" are used as synonymous and mean any component that is intended to furnish pharmacological activity or other direct effect in the diagnosis, cure, mitigation, treatment, or prevention of disease, or to affect the structure or any function of the body of man or other animals.

The terms "iron overload" or "overload of iron" are used interchangeably herein and refer to medical conditions where the body contains or stores too much (or "excess") iron. An example is transfusional iron overload, where the excess iron is introduced by one or more blood transfusions.

The term "minitablets" commonly refers to compressed tablets with size smaller than typical tablets. Although there are currently no regulatory guidelines defining minitablets (sometimes referred to as microtablets), the term has been used to describe tablets with diameters between one to four millimeters.

With the term "glyceryl esters of long fatty acids" is meant a substance wherein one two, or three alcoholic groups of the glycerol moiety are esterified with long chain saturated fatty acids C₁₄-C_{22,} and mono-, di-, triglycerides are formed or mixture thereof.

In the present context, the term "hydrophilic" describes a molecule or portion of a molecule which is typically electrically polarized and capable of forming hydrogen bonds with water molecules, enabling it dissolve more readily in water than in oil or other "non-polar" solvents.

Conversely, the term "hydrophobic" denotes a compound tending to be electrically neutral and non-polar, and thus preferring other neutral and nonpolar solvents or molecular environments.

In the present context, the term "amphiphilic" describes a molecule having a polar watersoluble group attached to a water-insoluble hydrocarbon chain. Thus, one end of the molecule is hydrophilic (polar) and the other is hydrophobic (non-polar).

For "pH dependent solubility" it is meant a substance having different solubilities at different pHs. These pH-dependent solubility differences lead to pH-dependent dissolution profiles.

The expression "insoluble or poorly water soluble" refers to a substance having a solubility in water as defined in the European Pharmacopoeia Ed. 4th, 2003, page 2891.

"Core" or "tablet core" as used herein comprises an active ingredient, e.g., deferiprone, and one or more excipients compressed into an uncoated tablet. The core can be coated with various coatings, including an enteric coating.

In the present context, the terms "controlled release", "prolonged release", "modified release" and "delayed release" are intended to be equivalent terms covering some types of release of deferiprone from a composition of the invention that is appropriate to obtain a specific therapeutic or prophylactic response after administration to a subject". The terms refer to protecting an active ingredient, e.g., deferiprone, from rapid release at acidic pH, e.g., in the stomach, while enabling the active ingredient to be released at a higher rate at a higher pH, e.g., in the intestines. In some aspects, DR will be understood to mean that, when tested in USP apparatus 2 at 75 rpm, the extent of dissolution will be around 20 ± 5% at 1 hour in 0.1N HC1, and the rate of dissolution will be substantially higher (e.g., over 30%, e.g. over 40%, in 1 hour) in phosphate buffer with pH 6.8 than the rate of dissolution in 0. 1 N HC1.

In alternative, when tested in a USP apparatus 1 at 100 rpm, the extent of dissolution will be below than 10% at 2 hours in 0.1N HC1, and the rate of dissolution will be substantially higher (e.g. over 40%, in 1 hour) in phosphate buffer with pH 6.8 than the rate of dissolution in 0. 1 N HC1.

"Disintegrant" as used herein refers to an excipient that is insoluble in water, but swells when wetted to cause a tablet to disintegrate.

"Dissolution" as used herein refers to the process by which a solute forms a solution in a solvent.

"Enteric coat" or "enteric coating" as used herein refers to a coating comprising an enteric polymer. An enteric coating can serve to prevent or delay a tablet's dissolution or disintegration in a gastric environment.

"Enteric coated tablet" means a tablet having a core comprising an active ingredient, which is coated with an enteric coating.

"Enteric polymer" as used herein is understood to mean a polymer that is relatively insoluble at the acidic pH of the fasted stomach (e.g., about pH 1 to about pH 4), but soluble at higher pH (e.g., about pH 4.5 to about pH 8), which corresponds to the pH in the small intestine or thereafter, particularly in the duodenum or ileum.

The term "plasticizer" means an additive that increase the elasticity of coatings based on film-forming material.

The terms "fillers", "diluents" and "bulking agents" are used as synonymous.

With the term "bioequivalence" it is meant the absence of a significant difference between the bioavailability, i.e., the extent of absorption and peak concentration, between two pharmaceutical drug products (e.g., a test product and a reference product) over the course of a period of time, at the same dose and under the same conditions.

The determination of whether or not a test product is bioequivalent to a reference product is determined by performing a study, referred to as a bioequivalence or comparative bioavailability study, in a group of subjects, usually about 18-36 subjects or more, under controlled conditions.

The study can be done in a "crossover" design, which means that the study is done in 2 or more phases, usually at least a week apart, depending in part on the half-life of the drug. In the first phase, half the subjects are randomly assigned to ingest the test product first and the other half ingest the reference product first. In the second phase, each subject ingests the alternate product.

In each phase, blood samples are drawn from each subject, on a predetermined schedule after ingestion of the test product. The blood samples are then analyzed to determine serum concentrations of the drug (test product, e.g., deferiprone) at each time point. For example, drugs are bioequivalent if they enter circulation at the same rate when given in similar doses under similar conditions. Parameters often used in bioequivalence studies are tₘₐₓ, Cₘₐₓ, Cₘᵢₙ, AUC_{0-infinity}, AUC₀₋ₜ.

In the present context "tₘₐₓ" denotes the time to reach the maximal plasma concentration (Cₘₐₓ) after administration; "AUC_{0-infinity}" denotes the area under the plasma concentration versus time curve from time 0 to infinity; "AUC₀₋ₜ" denotes the area under the plasma concentration versus time curve from time 0 to time t; "W50" denotes the time where the plasma concentration is 50% or more of Cₘₐₓ; "W75" denotes the time where the plasma concentration is 75% or more of Cₘₐₓ; and "MRT" denotes mean residence time for tacrolimus.

"Fasted state" as used herein refers to abstinence from food for a defined period after a meal (typically, at least several hours, e.g., 4 or 6 hours, after a meal).

"Fed state" as used herein refers to administration with a meal or soon after a meal (e.g., within about 1 hour).

The term "chemical stable" refers to stability of the active agent in the formulation, wherein changes in the drug assay values and/or impurities content are equal to or lesser than 5%, preferably lesser than 3%, during storage at 25°C and 60% relative humidity (RH), or 40°C and/or 75% RH, for at least 1 month.

The term "in vitro-in vivo" correlation (IVIVC) refers to an in vitro dissolution test that is predictive of the in vivo performance of the drug product.

"Gastric distress" as used herein refers to discomfort of the gastrointestinal (GI) tract, e.g., one or more of pain, cramping, bloating, nausea, indigestion, heartburn, and gas.

"Tablet" as used herein refers a solid oral pharmaceutical dosage form.

"Half tablet" as used herein means either of the two parts of a tablet obtained by splitting the tablet into two parts of equal or approximately equal weight.

"Percent" or "%" as used herein refers to weight percentage (w/w) unless otherwise specified.

"Scored tablet" as used herein refers to a tablet that is debossed with one or more lines, also known as a "score line", to facilitate splitting the tablet, e.g., to enable administration of a half tablet.

"Whole tablet" means a complete tablet, i.e., not broken or split into parts.

Terms such as "treating" or "treatment" or "to treat" or "ameliorating" or "alleviating" or "to alleviate" can refer to both 1) therapeutic measures that cure, slow down, lessen symptoms of, reverse, and/or halt progression of a diagnosed pathologic condition or disorder and 2) prophylactic or preventative measures that prevent, reduce the incidence of, reduce the risk of, and/or slow the development of a targeted pathologic condition or disorder. Thus, those in need of treatment include those who already have the disorder; those prone to developing the disorder; and those in whom the disorder is to be prevented. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those who already have the condition or disorder as well as those prone to developing the condition or disorder or those in which the condition or disorder is to be prevented or incidence reduced.

By "subject" or "individual" or "patient," is meant any human subject, for whom diagnosis, prognosis, treatment, or therapy is desired.

By "therapeutically effective dose or amount" or "effective amount" is intended an amount of active pharmaceutical ingredient, e.g., deferiprone, that when administered brings about a positive therapeutic response with respect to treatment or reduces the risk of a disease in a subject to be treated.

It will be understood that the deferiprone DR tablets used as the "reference" or "Reference Product" herein are Ferriprox^{®} tablets (1000 mg) as approved by FDA and sold in the United States.

### DETAILED DESCRIPTION OF THE INVENTION

The present invention concerns pharmaceutical formulations for the prevention and/or treatment of diseases which are caused by an overload of iron, especially compositions providing modified release of the active ingredient.

The active ingredient in the inventive formulations is deferiprone.

However, within the scope of the present invention is deferiprone in any physical form (crystals, amorphous powder, any possible polymorphs, any possible solvate). Included are also pharmaceutically acceptable salts and/or solvates thereof. Preferably, deferiprone is used as a base in its anhydrous form.

In a first aspect, the invention is directed to a modified release enteric coated pharmaceutical formulation in form of minitablets for twice-a-day oral administration, wherein the core of the minitablet comprises deferiprone as active ingredient in an amount comprised between 74% and 87%, a glyceryl ester of a long fatty acid as modifying release agent in an amount comprised between 10 and 20%, a lubricant and/or glidant in an amount of 3 to 6%, and optionally another excipient in an amount of 0 to 2%, all the amounts calculated by weight on the total weight of the uncoated formulation, wherein the enteric coating comprises a mixture of methacrylic acid - ethyl acrylate copolymer (1:1), a plasticizer, and an emulsifier.

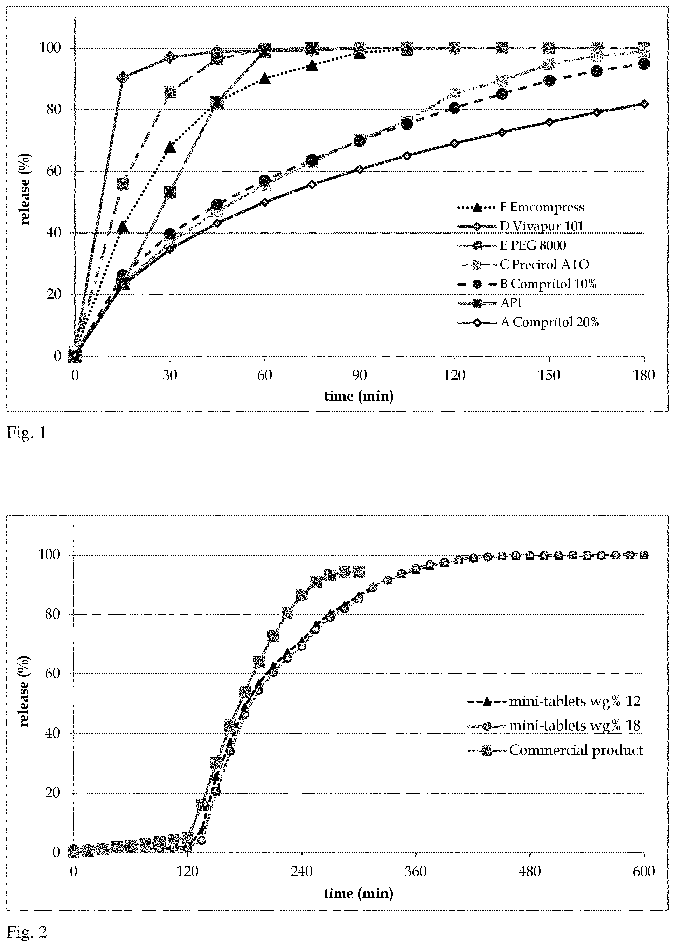

It has been found that, within the limits of the statistical significance, the formulation of the invention is able of providing an in vitro dissolution profile similar to commercial deferiprone DR tablets. This is shown in Figure 2.

Since it has been established that for Ferriprox^{®} tablets, that deferiprone modified release formulations exhibit a good IVIV correlation (WO 2019/082128), it is contemplated that the in vitro release profile will reflect the in vivo behaviour, so it is contemplated that the formulation of the invention will show the same bioavailability at the steady state, making it suitable for a twice a day oral administration.

It also follows that said the formulation of the invention would turn out to be bioequivalent in the steady state, to the immediate release Ferriprox^{®} tablets for three times a day administration, the mean ratio of AUC (over 24 hours) and the mean ratio of Cₘₐₓ for the tablets of the invention relative to the immediate release (IR) tablets would be within 80% to 125%.

In other words, it is plausible that, at the steady state, the modified release tablets of the present invention when administered twice-a-day would be able to achieve a similar maximum peak concentration (Cmax) as IR tablets of Ferriprox^{®}, when the IR tablets were given three times a day, and the total amount absorbed (AUC) would be similar for both products over a 24-hour period.

On the other hand, the formulation of the invention would overcome the problems related to the administration of the half tablets, i.e. the partial destruction of the enteric coating.

In fact, it would be possible to administer the desired dose by simply counting the number of necessary minitablets which in turn would maintain the entire coating, so guaranteeing a higher dosage flexibility.

Furthermore, the formulation of the present invention has the advantage of having a composition based on excipients with non-pH-dependent solubility, and hence they are not directly interfering with the release of deferiprone at the different pH values of the gastrointestinal tract. This would substantiate an undeniable improvement in terms of reproducibility of the expected release profiles.

Advantageously, the glyceryl esters of long fatty acids are selected from the group consisting of glyceryl palmitostearate, glyceryl monostearate and glyceryl dibehenate.

Glyceryl dibehenate, also known as Compritol^{®} 888 ATO, is a water-insoluble mixture of glyceryl esters of behenic acid commercially available from Gattefossé SAS, Saint-Priest Cedex, France. In particular, it is a mixture of mono-, di- and triglycerides, the di-ester being the predominant form (40-60% by weight).

Glyceryl palmitostearate is also known as Precirol^{®} 5 ATO and is commercially available from Gattefossé SAS, Saint-Priest Cedex, France as well.

Glyceryl monostearate is a monoglyceride commercially available from Sigma Aldrich GmbH (Germany).

In a preferred embodiment, Compritol^{®} 888 ATO or Precirol^{®} 5 ATO is used.

In fact, as it can be appreciated from Figure 1, Compritol^{®} 888 ATO or Precirol^{®} 5 ATO gives rise to a similar dissolution profile.

In an even more preferred embodiment of the invention, Compritol^{®} 888 ATO is used.

The amount of the glyceryl ester of long fatty acids shall be comprised between 10 and 20% based on the total amount of the formulation, preferably from 10 to 15%, more preferably of 10%.

In fact, it was found that contrary to what reported in the co-pending application n. EP22167557.2, wherein an amount of Compritol^{®} of about 10% is considered suitable for once-a-day administration while lower amounts are suitable for twice-a-day administration, with the minitablets of the invention higher amounts are necessary to provide a dissolution suitable for twice-a-day administration

Without being limited by the theory, this could be since minitablets with an equal mass are endowed with a greater surface area than standard tablets.

Deferiprone may cause gastric irritation if released in the fasted stomach, and some degradation by acidic hydrolysis.

Therefore, the coating shall be able of giving rise to a neglectable dissolution in the stomach.

This is achieved by using as enteric coating comprising methacrylic acid - ethyl acrylate copolymer (1:1).

The methacrylic acid - ethyl acrylate copolymer (1:1) is known as Eudragit^{®} L30-D55 and is commercially available from Evonik Operations GmbH, Essen Germany.

The enteric coating may comprise, in addition to said enteric polymer, other excipients such a plasticizer, a lubricant or anti-tack agent, an opacifier, a colorant, a diluent, or any combination thereof.

Advantageously, the enteric coating plasticizer is diethyl phthalate, citrate esters such as triethyl citrate (TEC), polyethylene glycol, glycerol, acetylated glycerides, acetylated citrate esters, dibutyl sebacate, castor oil, or any combination thereof, preferably triethyl citrate.

The enteric coating may further comprise an emulsifier and a co-emulsifier or a mixture thereof. Advantageously, the emulsifier could be selected from the group comprising, but not limited to, sorbitan monolaurate, polysorbate 20 (known as Tween 20^{®}), polysorbate 80 (known as Tween 80^{®}), preferably polysorbate 80, while the co-emulsifier could be selected from cetyl alcohol, cetearyl alcohol, sucrose stearate and glyceryl monostearate, preferably glyceryl monostearate (GMS).

Advantageously, the components of the enteric coating are present in the following percentages: 90-95% enteric polymer, 1.0-3.0% plasticizer, 2.0-4.0% emulsifier or mixture thereof with the co-emulsifier.

In a preferred embodiment, the enteric coating comprises methacrylic acid - ethyl acrylate copolymer (1:1), triethyl citrate, polysorbate 80 and glyceryl monostearate.

The coating shall be performed according to methods known to the skilled person.

The minitablets of the invention could also comprise a lubricant to prevent sticking to the tooling during compression into tablets, and/or a glidant to improve flow in the tableting process, or combinations thereof in an amount of 1.0 to-6.0% calculated based on the weight of the uncoated formulation, preferably from 3.0 to 5.0% by weight.

Advantageously, the lubricant is selected from the group consisting of, but not limited to, magnesium stearate, calcium stearate, stearic acid, sodium stearyl fumarate, or combination thereof.

Advantageously, the glidant is selected from the group consisting of, but not limited to, colloidal silicon dioxide, starch and talc, preferably colloidal silicon dioxide or combination thereof.

In a preferred embodiment, the core of the minitablets comprises 5% talc and 0.5% magnesium stearate.

In some embodiments, the core of the minitablet may comprise one or more pharmaceutically acceptable excipients such as bulking agents.

Advantageously, the bulking agent that when present is utilizes to increase tablet hardness, could be selected from the group consisting of calcium carbonate, dibasic calcium phosphate, tribasic calcium phosphate, calcium sulfate, microcrystalline cellulose, powdered cellulose, dextrans, dextrins, dextrose, fructose, kaolin, lactose, mannitol, sorbitol, starch, pregelatinized starch, sucrose, alpha-lactose monohydrate.

In particular embodiments, the minitablets could also comprise a basic excipient selected from the group consisting of meglumine, metal oxides, metal hydroxides, basic salts of weak acids, and a combination thereof. Metal oxides include, but are not limited to, magnesium oxide, aluminum oxide, and zinc oxide. Metal hydroxides include, but are not limited to, sodium hydroxide, potassium hydroxide, magnesium hydroxide, and calcium hydroxide. Basic salts of weak acids include, but are not limited to, sodium or potassium salts of carbonate, bicarbonate, acetate, and citrate. In certain embodiments, the basic excipient is magnesium oxide, meglumine or a combination thereof. In some embodiments, the basic excipient is magnesium oxide.

However, preferably the minitablets of the invention do not contain a basic excipient.

As a preferred embodiment, of the core of the coated minitablets of the invention comprises:
i) deferiprone in an amount of 84.5% by weight; glyceryl behenate in an amount of 10 % by weight;
ii) talc in an amount of 5.0% by weight;
iii) magnesium stearate in an amount of 0.5% by weight.

Preferably, the coating has the following composition:

| **Coating composition** | % |
|---|---|
| Eudragit^{®} L30 D-55 | 94.1 |
| Triethyl citrate (TEC) | 2.8 |
| Tween^{®} 80 | 1.7 |
| Glyceryl monostearate GMS) | 1.4 |

The person skilled in the art shall properly adjust the thickness of the coating to make to dissolution profile of the minitablets similar to the reference formulation.

Typically, the thickness of the coating is of 20-40 micron.

Said thickness corresponds to an increment in weigh of 10 to 20%, preferably 12 to 18%. Otherwise, the thickness, expressed as amount of polymer for surface unit to values, could be comprised between 4 and 10 mg/cm².

The release profile of the tablets of the invention has been determined in different dissolution media varying the pH according to the conditions reported in Example 1.

Typically, as a result of the dissolution test, no significant release was observed at pH 1.2, while, upon change, at pH 6.8 the formulation of the invention releases less than 50% w/v of the active ingredient within 1.0 hours, equal or less than 85% w/v within 3 hours, equal or less than 95% w/v within 4 hours.

The invention also provides a process for the preparation of the coated deferiprone minitablets as described above, said process comprising:
i) mixing deferiprone with the modifying release agent and the optional excipients, if present, to form a mixture;
ii) tableting the mixture of step i) to obtain compacts; crushing the compacts through a granulator with a suitable screen size to obtain a granulate;
iii) mixing the granulate obtained in step (iii) with the lubricant/glidant excipient to form a final mixture;
iv) compressing the final mixture obtained in step (iv) to form a minitablet; and
v) coating and drying the minitablets.

Apparatus and conditions for compaction and compression upon granulation are known to the skilled person in the art. Therefore, the operating parameters shall be adjusted according to its knowledge.

For example, typical parameters are reported in Table 3 of Example 1.

Typically, the minitablets have a diameter of 2.5-3.0 mm, preferably 2.6-2.7 mm, and a height of 2.2-2.3 mm.

Apparatus and conditions for drying the coated minitablets are known in the art.

The present disclosure provides dosing regimens useful for the therapeutic use of the pharmaceutical formulations described herein.

Typically, the oral daily dose of deferiprone could range from 75 mg/kg to 100 mg/kg.

The solid unit dose of deferiprone is typically 1000 mg, but depending on the number of minitablets, different doses could be administered according to the sex, age, weight of the patient.

The claimed formulations are useful for the treatment of diseases which cause an overload of iron, or for the prevention and/or treatment of diseases which are caused by an overload of iron.

In some embodiments, the subject in need thereof suffers from iron overload due to transfusional iron overload, or due to diseases such as thalassemia, myelodysplasia, or sickle cell disease.

In some embodiments, the subject in need thereof could suffer from a neurodegenerative disease (e.g., Parkinson's disease, amyotrophic lateral sclerosis (ALS), Huntington's disease, Friedreich's Ataxia, Pantothenate Kinase Associated Neurodegeneration (PKAN), or neurodegeneration with brain iron accumulation (NBIA).

In some embodiments, the subject in need thereof suffers from iron overload that is transfusional iron overload.

In certain aspects, the subject suffers from transfusional iron overload and whose prior chelation therapy is inadequate. In certain aspects, the subject suffers from transfusion iron overload and has a cardiac MRI T2* of 20 ms or less (e.g., 10 ms).

The invention is also directed to a container filled with the disclosed minitablets.

For example, typical containers are capsules or sachets.

The invention is illustrated in detail by the following examples.

### EXAMPLES

### Example 1 - Preparation of uncoated minitablets and relevant characterization

Minitablets containing deferiprone were prepared by tableting dry granules obtained by slugging.

Dry granulation. Mixtures with composition reported in Table 1 except for magnesium stearate were tableted into cylindrical compacts in a rotary tablet press (Officine Meccaniche Ronchi, AM8S) equipped with 11 mm diameter flat punches. Compaction force (Fa) was set to have tablets with a crushing strength of about 40 N. Compacts were crushed in an oscillating granulator (Erweka, Wet granulator FGS) with a screen size of 1000 µm. Excipients proposed were evaluated according to their typical applications in pharmaceutical formulations: Compritol 888 ATO and Precirol ATO were used as the controlling release materials; Vivapur, PEG 8000, Emcompress as the diluents/fillers; talc as the antitacking agent; magnesium stearate as the lubricant in tableting.

**Table 1. Formulations and properties of uncoated minitablets.**

| **Formulation** | **Composition (% w/w)** | | | | | | | | **Tablet properties** | |
|---|---|---|---|---|---|---|---|---|---|---|
| | API | Compritol^{®} 888 ATO | Precirol^{®} ATO | Vivapur 101 | PEG 8000 | Emcompress^{®} | Talc | Mg stearate | Weight* (mg) | Friability (%) |
| A | 74.63 | 19.9 | - | - | - | - | 4.97 | 0.5 | 12.45±0.59 | 0.55 |
| B | 84.58 | 9.95 | - | - | - | - | 4.97 | 0.5 | 12.56±0.64 | 0.61 |
| C | 84.58 | - | 9.95 | - | - | - | 4.97 | 0.5 | 12.68±0.62 | 0.52 |
| D | 89.55 | - | - | 9.95 | - | - | - | 0.5 | 11.88±0.51 | 0.85 |
| E | 89.55 | - | - | - | 9.95 | - | - | 0.5 | 12.87±0.26 | 0.29 |
| F | 89.55 | - | - | - | - | 9.95 | - | 0.5 | 12.71±0.42 | 0.35 |

| | | | | | | | | | | |
|---|---|---|---|---|---|---|---|---|---|---|
| *mean ±sd n=20 | | | | | | | | | | |

Tableting. Granules were added with 0.5% of magnesium stearate and compacted into mini tablets by means of the same press, equipped with concave punches (diameter 2.5 mm, curvature radius 2.5 mm). Compression force was set at 1 kN. The minitablets produced had a mass of approximately 12 mg, height of 2.2 mm and friability <1% (Table 1).

Friability was determined according to the method reported in the Ph Eur 10^{th} Ed.

Dissolution test (uncoated minitablets). Dissolution tests were carried out in Apparatus 2 in 900 ml of pH 6.8 medium, at paddle rotation speed of 50 rpm. Minitablet sample mass corresponding to 1000 mg of deferiprone was tested in triplicate. Dissolution profiles of formulations A-F along with that of unformulated drug are presented in Figure 1.

As it can be appreciated, formulations comprising Compritol^{®} and Precirol^{®}, due to their lipophilic properties, exhibit similar dissolution profile.

Formulation B (Compritol^{®} 888 ATO 10% w/w) were selected to be coated with a gastro-resistant film.

### Example 2 - Preparation of coated minitablets and relevant characterization

Coating was performed in fluid bed equipment provided with Wurster insert (Mini-Glatt, Glatt GmbH, D). Coating suspension (composition in Table 2) was applied under the experimental conditions reported in Table 3.

The resulting coated minitablets were dried.

**Table 2. Composition of enteric coating formulation.**

| **Coating composition** | **%** |
|---|---|
| Eudragit^{®} L30 D-55 | 94.1 |
| Triethyl citrate (TEC) | 2.8 |
| Tween^{®} 80 | 1.7 |
| glyceryl monostearate (GMS) | 1.4 |

**Table 3. Coating process parameters.**

| **Parameters** | | |
|---|---|---|
| Batch size | 50 | g |
| Inlet air temperature | 30 | °C |
| Product temperature | 26-30 | °C |
| Coater airflow | 30 | m³/hr |
| Atomization pressure | 1 | bar |
| Liquid spraying rate | 1.96 | g/min |

In order to prepare units at different level of coating, samples were collected after 27 minutes (batch 1) and 41 minutes (batch 2) during the process, corresponding to weight gain of 11.92 and 17.89% respectively, as calculated according to the formula: $w . g . % = \frac{{m}_{coated} - {m}_{uncoated}}{{m}_{uncoated}} \times 100$

Units were characterized for height and diameter using a digital micrometer (Absolute, Mitutoyo, J, n=20); thickness of the coating layer was calculated using the following formula: $s = \frac{\frac{{h}_{coated} - {h}_{uncoated}}{2} + \frac{{d}_{coated} - {d}_{uncoated}}{2}}{2} * 1000$

**Table 4. Properties of enteric coated minitablets**

| | **Batch 1** | **Batch 2** |
|---|---|---|
| Weight gain (%) | 11.92 | 17.89 |
| Coating amount per unit area (mg/cm²) | 6.66 | 9.99 |
| Tablet height (mm) | 2.254 ± 0.112 | 2.262 ± 0.098 |
| Tablet diameter (mm) | 2.627 ± 0.048 | 2.677 ± 0.031 |
| Coating layer thickness (µm) | 46.74 ± 34.36 | 61.39 ± 27.87 |

### Dissolution test of the coated minitablets

Tests were performed in Apparatus 2 at 37 ° and paddle rotation speed of 50 rpm, according to "Delayed-release solid dosage forms, method B" of Ph.Eur. 10^{th} Ed.

The spectrum of maximum absorption of the active was acquired in the various fluids in which the release tests was conducted by means of a spectrophotometer.

Compositions of dissolution media are reported below.
pH 1.2: for 1 L, 3.73 g KCI, 7.07 ml HCI 1N (deionized water up to volume) pH 4.5: for 1 L, 6.80 g of KH₂P0₄ (deionized water up to volume)
pH 6.8: for 1 L, 6.80 g KH₂P0₄, 0.90 g of NaOH (deionized water up to volume)
Calibration curves were built for each of the release media both at the wavelength of 276 nm at which a peak of absorbance was recorded, and at 243nm, in which reduced absorption was observed, in order not to exceed the instrument maximum absorbance value.

The release test of the commercial product was analyzed at the wavelength of 276 nm with the pH change mode (HCI 0.1 N for the first 120 minutes and phosphate buffer pH 6.8 for the remainder of the test).

Dissolution profiles of coated minitablet at different coating level along with that of reference commercial product are presented in Figure 2.

As it can appreciated from Figure 2, within the limits of the statistical significance, the formulation of the invention is able of providing an in vitro dissolution profile similar to commercial deferiprone DR tablets (Reference Product).

In particular, up to 240 minutes, the two profiles are substantially overlappable.

## Claims

1. A modified release enteric coated pharmaceutical formulation, in form of minitablets for twice-a-day oral administration, wherein the core of the tablet comprises deferiprone as active ingredient in an amount comprised between 74% and 87%, a glyceryl ester of a long fatty acid as modifying release agent in an amount comprised between 10% and 20%, a lubricant and/or glidant in an amount of 1 to 6%, and optionally another excipient in an amount of 0 to 2%, all the amounts calculated by weight on the total weight of the uncoated formulation, wherein the enteric coating comprises a mixture of methacrylic acid - ethyl acrylate copolymer (1:1), a plasticizer, and an emulsifier.

2. The pharmaceutical formulation according to claim 1, wherein the glyceryl esters of long fatty acids agents are selected from the group consisting of glyceryl palmitostearate, glyceryl monostearate and glyceryl dibehenate.

3. The pharmaceutical formulation according to claim 1 or 2, wherein the glyceryl esters of long fatty acids agent is glyceryl dibehenate.

4. The pharmaceutical formulation according to any one of claims 1 to 3, wherein the lubricant is selected from the group consisting of magnesium stearate, calcium stearate, stearic acid, sodium stearyl fumarate, talc, and a combination thereof.

5. The pharmaceutical formulation according to claim 4, wherein the lubricant is magnesium stearate.

6. The pharmaceutical formulation according to any one of claims 1 to 5, wherein the glidant is selected from the group consisting of colloidal silicon dioxide, starch and talc and combination thereof.

7. The pharmaceutical formulation according to claim 6, wherein the glidant is talc.

8. The pharmaceutical formulation according to any one of the preceding claims, wherein the other suitable pharmaceutically acceptable excipients, if present, are selected from the class of bulking agents.

9. The pharmaceutical formulation according to any one of preceding claims, wherein the plasticizer is triethyl citrate.

10. The pharmaceutical formulation according to any one of the preceding claims wherein the emulsifier is selected from the group comprising, but not limited to, sorbitan monolaurate, polysorbate 20 (Tween 20^{®}), and polysorbate 80 (Tween 80^{®}).

11. The pharmaceutical formulation according to claim 10, wherein the emulsifier is polysorbate 80.

12. The pharmaceutical formulation according to any one of the preceding claims, wherein the coating further comprises a co-emulsifier selected from the group consisting of cetyl alcohol, cetearyl alcohol and sucrose stearate.

13. The pharmaceutical formulation according to claim 12, wherein the co-emulsifier is glyceryl monostearate.

14. A process for the preparation of the pharmaceutical formulation according to any one of the preceding claims, said process comprising the following steps:
(i) mixing deferiprone with the modifying release agent and the optional excipients, if present, to form a mixture;
(ii) tableting the mixture of step i) to obtain compacts;
(iii) crushing the compacts through a granulator with a suitable screen size to obtain a granulate;
(iv) mixing the granulate obtained in step (iii) with the lubricant/glidant excipient to form a final mixture;
(v) compressing the final mixture obtained in step (iv) to form a minitablet;
(vi) coating the minitablet and
(vii) drying the coated minitablet.

15. The pharmaceutical formulation according to any one of claims 1 to 13, for use for the treatment of diseases which cause an overload of iron, or for the prevention and/or treatment of diseases which are caused by an overload of iron.

16. The pharmaceutical formulation for use according to claim 15, wherein the disease is thalassemia or sickle cell anemia.

17. The pharmaceutical formulation for use according to claim 15, wherein said iron overload is transfusional iron overload.

## Patentansprüche

1. Magensaftresistent beschichtete pharmazeutische Formulierung mit modifizierter Freisetzung in Form von Minitabletten zur zweimal täglichen oralen Verabreichung, wobei der Kern der Tablette Deferipron als Wirkstoff in einer Menge, die zwischen 74 % und 87 % liegt, einen Glycerylester einer langen Fettsäure als Mittel zur modifizierten Freisetzung in einer Menge, die zwischen 10 % und 20 % liegt, ein Gleitmittel und/oder Fließregulierungsmittel in einer Menge von 1 bis 6 % und gegebenenfalls einen weiteren Hilfsstoff in einer Menge von 0 bis 2 % umfasst, wobei alle Mengen nach Gewicht bezogen auf das Gesamtgewicht der unbeschichteten Formulierung berechnet sind, wobei die magensaftresistente Beschichtung eine Mischung aus Methacrylsäure-Ethylacrylat-Copolymer (1:1), einem Weichmacher und einem Emulgator umfasst.

2. Pharmazeutische Formulierung nach Anspruch 1, wobei die Mittel aus Glycerylestern langer Fettsäuren ausgewählt sind aus der Gruppe bestehend aus Glycerylpalmitostearat, Glycerylmonostearat und Glyceryldibehenat.

3. Pharmazeutische Formulierung nach Anspruch 1 oder 2, wobei das Mittel aus Glycerylestern langer Fettsäuren Glyceryldibehenat ist.

4. Pharmazeutische Formulierung nach einem der Ansprüche 1 bis 3, wobei das Gleitmittel ausgewählt ist aus der Gruppe bestehend aus Magnesiumstearat, Calciumstearat, Stearinsäure, Natriumstearylfumarat, Talkum und einer Kombination davon.

5. Pharmazeutische Formulierung nach Anspruch 4, wobei das Gleitmittel Magnesiumstearat ist.

6. Pharmazeutische Formulierung nach einem der Ansprüche 1 bis 5, wobei das Fließregulierungsmittel ausgewählt ist aus der Gruppe bestehend aus kolloidalem Siliciumdioxid, Stärke und Talkum und einer Kombination davon.

7. Pharmazeutische Formulierung nach Anspruch 6, wobei das Fließregulierungsmittel Talkum ist.

8. Pharmazeutische Formulierung nach einem der vorstehenden Ansprüche, wobei die anderen geeigneten pharmazeutisch verträglichen Hilfsstoffe, falls vorhanden, aus der Klasse der Füllstoffe ausgewählt sind.

9. Pharmazeutische Formulierung nach einem der vorstehenden Ansprüche, wobei der Weichmacher Triethylcitrat ist.

10. Pharmazeutische Formulierung nach einem der vorstehenden Ansprüche, wobei der Emulgator ausgewählt ist aus der Gruppe umfassend, aber nicht beschränkt auf, Sorbitanmonolaurat, Polysorbat 20 (Tween 20^{®}) und Polysorbat 80 (Tween 80^{®}).

11. Pharmazeutische Formulierung nach Anspruch 10, wobei der Emulgator Polysorbat 80 ist.

12. Pharmazeutische Formulierung nach einem der vorstehenden Ansprüche, wobei die Beschichtung ferner einen Co-Emulgator umfasst, ausgewählt aus der Gruppe bestehend aus Cetylalkohol, Cetearylalkohol und Saccharosestearat.

13. Pharmazeutische Formulierung nach Anspruch 12, wobei der Co-Emulgator Glycerylmonostearat ist.

14. Prozess zur Herstellung der pharmazeutischen Formulierung nach einem der vorstehenden Ansprüche, wobei der Prozess die folgenden Schritte umfasst:
(i) Mischen von Deferipron mit dem die Freisetzung modifizierenden Mittel und den optionalen Hilfsstoffen, falls vorhanden, zur Bildung einer Mischung;
(ii) Tablettieren der Mischung aus Schritt i), um Presslinge zu erhalten;
(iii) Zerkleinern der Presslinge durch einen Granulator mit einer geeigneten Siebgröße, um ein Granulat zu erhalten;
(iv) Mischen des in Schritt (iii) erhaltenen Granulats mit dem Gleitmittel- /Fließregulierungsmittel-Hilfsstoff zur Bildung einer Endmischung;
(v) Komprimieren der in Schritt (iv) erhaltenen Endmischung zur Bildung einer Minitablette;
(vi) Beschichtung der Minitablette und
(vii) Trocknen der beschichteten Minitablette.

15. Pharmazeutische Formulierung nach einem der Ansprüche 1 bis 13 zur Verwendung für die Behandlung von Erkrankungen, die eine Eisenüberladung verursachen, oder für die Prävention und/oder Behandlung von Erkrankungen, die durch eine Eisenüberladung verursacht werden.

16. Pharmazeutische Formulierung zur Verwendung nach Anspruch 15, wobei die Erkrankung Thalassämie oder Sichelzellanämie ist.

17. Pharmazeutische Formulierung zur Verwendung nach Anspruch 15, wobei die Eisenüberladung eine transfusionsbedingte Eisenüberladung ist.

## Revendications

1. Formulation pharmaceutique à libération modifiée à enrobage entérique, sous forme de minicomprimés pour une administration orale deux fois par jour, dans laquelle le noyau du comprimé comprend de la défériprone en tant que principe actif en une quantité comprise entre 74 % et 87 %, un ester de glycéryle d'un acide gras long en tant qu'agent de modification de la libération en une quantité comprise entre 10 % et 20 %, un lubrifiant et/ou un agent de glissement en une quantité de 1 à 6 %, et facultativement un autre excipient en une quantité de 0 à 2 %, toutes les quantités étant calculées en poids par rapport au poids total de la formulation non enrobée, dans laquelle l'enrobage entérique comprend un mélange de copolymère d'acide méthacrylique-acrylate d'éthyle (1:1), un plastifiant et un émulsifiant.

2. Formulation pharmaceutique selon la revendication 1, dans laquelle les agents esters de glycéryle d'acides gras longs sont choisis dans le groupe constitué par le palmitostéarate de glycéryle, le monostéarate de glycéryle et le dibéhénate de glycéryle.

3. Formulation pharmaceutique selon la revendication 1 ou 2, dans laquelle l'agent d'esters de glycéryle d'acides gras longs est le dibéhénate de glycéryle.

4. Formulation pharmaceutique selon l'une quelconque des revendications 1 à 3, dans laquelle le lubrifiant est choisi dans le groupe constitué par le stéarate de magnésium, le stéarate de calcium, l'acide stéarique, le fumarate de stéaryle de sodium, le talc, et une combinaison de ceux-ci.

5. Formulation pharmaceutique selon la revendication 4, dans laquelle le lubrifiant est le stéarate de magnésium.

6. Formulation pharmaceutique selon l'une quelconque des revendications 1 à 5, dans laquelle l'agent de glissement est choisi dans le groupe constitué par le dioxyde de silicium colloïdal, l'amidon et le talc et une combinaison de ceux-ci.

7. Formulation pharmaceutique selon la revendication 6, dans laquelle le glissant est du talc.

8. Formulation pharmaceutique selon l'une quelconque des revendications précédentes, dans laquelle les autres excipients pharmaceutiquement acceptables appropriés, s'ils sont présents, sont choisis dans la classe des agents de charge.

9. Formulation pharmaceutique selon l'une quelconque des revendications précédentes, dans laquelle le plastifiant est le citrate de triéthyle.

10. Formulation pharmaceutique selon l'une quelconque des revendications précédentes, dans laquelle l'émulsifiant est choisi dans le groupe comprenant, sans s'y limiter, le monolaurate de sorbitane, le polysorbate 20 (Tween 20^{®}) et le polysorbate 80 (Tween 80^{®}).

11. Formulation pharmaceutique selon la revendication 10, dans laquelle l'émulsifiant est le polysorbate 80.

12. Formulation pharmaceutique selon l'une quelconque des revendications précédentes, dans laquelle l'enrobage comprend en outre un co-émulsifiant choisi dans le groupe constitué par l'alcool cétylique, l'alcool cétéarylique et le stéarate de saccharose.

13. Formulation pharmaceutique selon la revendication 12, dans laquelle le co-émulsifiant est le monostéarate de glycéryle.

14. Procédé de préparation de la formulation pharmaceutique selon l'une quelconque des revendications précédentes, ledit procédé comprenant les étapes suivantes :
(i) le mélange de la défériprone avec l'agent modifiant la libération et les excipients facultatifs, s'ils sont présents, pour former un mélange ;
(ii) la compression du mélange de l'étape i) pour obtenir des compacts ;
(iii) le broyage des compactés à travers un granulateur avec une taille de tamis appropriée pour obtenir un granulat ;
(iv) le mélange du granulé obtenu à l'étape (iii) avec l'excipient lubrifiant/agent de glissement pour former un mélange final ;
(v) la compression du mélange final obtenu à l'étape (iv) pour former un minicomprimé ;
(vi) l'enrobage du minicomprimé et
(vii) le séchage du minicomprimé enrobé.

15. Formulation pharmaceutique selon l'une quelconque des revendications 1 à 13, pour utilisation pour le traitement de maladies qui provoquent une surcharge en fer, ou pour la prévention et/ou le traitement de maladies qui sont causées par une surcharge en fer.

16. Formulation pharmaceutique pour utilisation selon la revendication 15, dans laquelle la maladie est la thalassémie ou la drépanocytose.

17. Formulation pharmaceutique pour utilisation selon la revendication 15, dans laquelle ladite surcharge en fer est une surcharge en fer transfusionnelle.
